# EUROPEAN PATENT APPLICATION

(11) **EP 2 921 176 A2**
(43) Date of publication of application: **23.09.2015**
(21) Application number: 13866485.9
(22) Date of filing: 14.11.2013
(51) Int. Cl.: A61K 38/06, A61K 9/00, A61K 9/12, A61K 9/19, A61K 9/14

(54) **PHARMACEUTICAL COMPOSITION FOR TREATMENT OF PROSTATIC HYPERPLASIA**

(30) Priority: 15.11.2012 EA 201201417
(71) Applicant: Zamerton Holdings Limited, 6018 Larnaka (CY)
(72) Inventor: OVCHINNIKOV, Mihail, Vladimirovich, Moscow 115162 (RU); CHERTORIZHSKIY, Evgeniy, Aleksandrovich, Obninsk Kaluzhskaya obl. 249031 (RU); BELY, Petr Aleksandrovich, Moscow 127055 (RU)
(74) Representative: Icosa
(86) International application number: PCT/IB2013/003050
(87) International publication number: WO 2014/106772

(57) **Abstract**

This invention relates to medicine and concerns a pharmaceutical composition containing the peptides Ala-Asp-Glu, Lys-Asp-Glu and Asp-Glu-Gly active in relation to benign prostatic hyperplasia, its application for treatment of benign prostatic hyperplasia, and the method of treating benign prostatic hyperplasia. The composition possesses a pronounced effect that is significantly superior to that of α₁-adrenergic blockers and inhibitors of 5 α-reductase. The discovered effect makes it possible to use the pharmaceutical composition containing the peptides Ala-Asp-Glu, Lys-Asp-Glu and Asp-Glu-Gly to treat benign prostatic hyperplasia.

## Description

### FIELD OF THE INVENTION

This invention is related to medicine and concerns a pharmaceutical composition containing tripeptides, its application for treatment of benign prostatic hyperplasia and a method of treating benign prostatic hyperplasia.

### DESCRIPTION OF THE PRIOR ART

Benign prostatic hyperplasia (BPH) is a condition caused by the enlargement of the periurethral zone of the prostate gland leading to obstruction of the lower urinary tract. BPH is one of the more common conditions in older men, and its incidence increases with age. By the age of 50 from 13% to 50% of men suffer from this condition, and at the age of 80 some 90% of men demonstrate morphological changes characteristic of BPH (O.B. Loran, I.V. Lukianov, Practical Urology: What Is New in the Treatment of BPH - An Expert's Perspective. Russian Medical Journal, 2008, No. 29, pp. 1988-1990; O.B. Loran, Naumov A.V., Vertkin A.L. et al. Benign Prostatic Hyperplasia and Its Complications in General Medical Practice. Handbook of Polyclinic Doctor. 2009, No. 11, pp. 3-7; O.I. Bratchikov, Ambarian A.A., Shumakova E.A. et al., Etiological and Prognostic Aspects of Renal Insufficiency in Patients with Prostatic Adenoma. Urology, 2010, No. 1, pp. 38-43; Elterman D.S., Barkin J., Kaplan S.A. Optimizing the management of prostatic hyperplasia. Ther. Adv. Urol., 2012, Vol. 4, No 2, p. 77-83). About half of such patients demonstrate macroscopic enlargement of the prostate gland, and 25% develop clinical pathological symptoms of the prostate that require treatment. The problem's social significance and relevance is emphasized by the WHO's demographic studies that show that the share of people above 60 is growing at a substantially faster rate than the total population on the planet. This trend is also present in our country.

Drug therapy makes it possible to reduce patients' complaints, improve impaired urination and create conditions for normalization of the quality of life, though it cannot cure BPH. Drug treatment is not recommended for patients with complicated BPH.

The main pharmaceuticals currently used for treatment of BPH, a ₁-adrenergic blockers (Doxazosin), 5 alpha-reductase inhibitors (Finasteride and Dutasteride), and extracts of *Serenoa repens* (saw palmetto) and *Pygeum africanum* (red stinkwood) are indicated only for initial and moderate symptoms of BPH (E.I. Veliev, Bogdanov A.B., Lukianov I.V. Effectiveness and safety of combined administration of doxazasin and finasteride for the treatment of prostatic adenoma. Urology, 2008, No. 6, pp. 44-48; O.B. Loran, I.V. Lukianov, Practical Urology: What Is New in the Treatment of BPH - An Expert's Perspective. Russian Medical Journal, 2008, No. 29, pp. 1988-1990; Tarasov, R.I. Izmailov, Tarasov N.I., Izmailov R.I. Possibilities of combined therapy with doxazosin and finasteride for treatment of patients with large prostatic adenoma. Urology, 2009, No. 5, pp. 40-45; Tkachuk V.N., Al-Shukri S.H., Tkachuk I.N., Mosoian M.S. Effectiveness and safety of combined application of 5α-reductase inhibitors and α-adrenergic blockers; O.I. Apolohin, Sivkov A.V., Beshliev D.A., Abdulin I.I. Current possibilities of pharmaceutical treatment of prostatic adenoma, Urology, 2010, No. 2, pp. 55-61; V.B. Borisov. On the treatment of prostatic adenoma. Urology, 2010, No. 4, pp. 42-44; Federal guidelines for use of pharmaceuticals (formulary system), Issue XII. Eds A.G.Chuchalin, Yu.B.Belousov, V.V. Yasnetsov - M.: «Ekho», 2011, p. 956; Cohen, Parsons, Combination pharmacological therapies for the management of benign prostatic hyperplasia, Drugs Aging, 2012, Vol. 29, No 4, p. 275-284; Elterman D.S., Barkin J., Kaplan S.A. Optimizing the management of benign prostatic hyperplasia, Ther. Adv. Urol., 2012, Vol. 4, No 2, p. 77-83; Goldfischer E., Kowalczyk J.J., Clark W.R. Hemodynamic Effects of Once-daily Tadalafil in Men With Signs and Symptoms of Benign Prostatic Hyperplasia on Concomitant α(1)-Adrenergic Antagonist Therapy: Results of a Multicenter Randomized, Double-Blind, Placebo-controlled Trial // Urology, 2012, Vol. 79, No 4, p. 875-882; Yoo T.K., Cho H.J. Benign prostatic hyperplasia: from bench to clinic // Korean J. Urol., 2012, Vol. 53, No 3, p. 139-148). The alpha 1-adrenergic blockers Alfuzosin, Doxazosin, Tamsulosin, Terazosin and Silodosin possess similar effectiveness. They can be reasonably used for symptoms of impaired urination due to BPH in the absence of absolute indications for surgery: chronic retention of the urinary tract, recurrent infections of the urinary tract, hematocyturia and others. Their effect develops after the first 2 to 3 weeks of administration.

Finasteride (which inhibits 5-alpha-reductase primarily of the 2nd type) affects the hormonal status and reduces the size of the prostate, but only after 6 months of uninterrupted therapy. It is more effective in cases of large BPH. Dutasteride is an inhibitor of isoenzymes of 5-alpha-reductase of the 1st and 2nd types, which explains its quicker therapeutic effect: reduction of the size of the prostate (more than 20%) after 1 to 3 months. The main side effects of the 5-alpha-reductase inhibitors are lower libido and erectile dysfunction.

The search for new safe and effective drug therapies for BPH is a pressing challenge for pharmacology and urology.

The peptide Ala-Asp-Glu and its influence of taste properties (Maechashi, Kenji et.al. "Isolation of peptides from enzymic hydtolyzate of food proteins and characterization of their taste properties". Bioscience, Biotechnology, and Biochemistry, 1999, 63 (3), 555-559) peptide Asp-Glu-Gly (Ji Jian Guo et.al. "Isolation and purification of a series of peptides from Panax notoginseng". Peptide: Biology and Chemistry. Proceedings of Chinese Peptides Symposium, 5th, Lanzhou, China, 1998, July 14-17, 102-103) are known, but they have never been considered for the treatment of conditions of the prostatic gland.

### SUMMARY

The authors of this invention are the first to propose a pharmaceutical composition that contains the peptides H-Ala-Asp Glu-OH, H-Lys-Asp-Glu-OH and H-Asp-Glu-Gly-OH as active ingredients in therapeutically effective quantities. The pharmaceutical composition proposed in accordance with the invention produces a pronounced effect on the benign prostatic hyperplasia and can be used for the treatment of this condition, in terms of the strength of its effect the pharmaceutical composition surpasses α₁-adrenergic blockers, 5 α-reductase inhibitors, and the homeopathic remedy Prostatine (OOO «Homeofarma», Russia, contains Pulsatilla C3, Serenoa (Sabal) C3, Staphisagria C3, Thuya C3, Zingiber C3).

Consequently, one of the aspects of this invention is a pharmaceutical composition containing as active ingredients the peptides H-Ala-Asp Glu-OH, H-Lys-Asp-Glu-OH and H-Asp-Glu-Gly-OH in therapeutically effective quantities.

According to the invention, the pharmaceutical composition in its preferred embodiment contains active ingredients in the following ratios: (1-8):(1-8):(1-8).

Another aspect of this invention is the use of the pharmaceutical composition containing such active components as the peptides H-Ala-Asp Glu-OH, H-Lys-Asp Glu-OH and H-Asp-Glu-Gly-OH in therapeutically effective quantities.

Another aspect of the invention is the method of treating the benign prostatic hyperplasia which provides for administration of the pharmaceutical composition containing as active components the peptides H-Ala-Asp-Glu-OH, H-Lys-Asp-Glu-OH 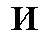 H-Asp-Glu-Gly-OH in therapeutically effective quantities.

### BRIEF DESCRIPTION OF THE FIGURES

Fig. 1. Prostates of intact rats after experiment.
Fig. 2. Light-optical changes in the prostate of control rats administered 10 mg/kg of testosterone propionate subcutaneously for 21 days.
Fig. 3. Light-optical changes in the prostate of rats with BPH injected intramuscularly with a dose of 150 mcg/kg/day of the composition according to the invention for 18 days.
Fig. 4. Light-optical changes in the prostate of rats with BPH injected intramuscularly with a dose of 600 mcg/kg/day of the composition according to the invention for 18 days.
Fig. 5. Light-optical changes in the prostate of rats with BPH who received Doxazosin into the stomach in a dose of 1 mg/kg/day for 18 days.
Fig. 6. Light-optical changes in the prostate of rats with BPH who received Finasteride into the stomach in a dose of 10 mg/kg/day for 18 days.
Fig. 7. Light-optical changes in the prostate of rats with BPH who received Prostatine in a dose of 5 pellets/day into the stomach for 18 days.

### DETAILED DESCRIPTION

This invention is the first to provide a pharmaceutical composition that is effective in regard to the benign prostatic hyperplasia and contains as active components the peptides H-Ala-Asp-Glu-OH, H-Lys-Asp-Glu-OH and H-Asp-Glu-Gly-OH in therapeutically effective quantities. The composition possesses a pronounced effect that is significantly stronger than that of α₁-adrenergic blockers and 5 α-reductase inhibitors. The discovered effect makes is possible to use the pharmaceutical composition containing the peptides H-Ala-Asp-Glu-OH, H-Lys-Asp-Glu-OH and H-Asp-Glu-Gly-OH for treatment of the benign prostatic hyperplasia.

In one of its aspects the invention is directed at a pharmaceutical composition containing as active components the peptides H-Ala-Asp Glu-OH, H-Lys-Asp-Glu-OH and H-Asp-Glu-Gly-OH in therapeutically effective quantities.

In the preferred embodiment the pharmaceutical composition according to the proposed invention contains the active components in the ratio of (1-8):(1-8):(1-8).

Another aspect of the invention is the use of a pharmaceutical composition containing the peptides H-Ala-Asp-Glu-OH, H-Lys-Asp-Glu-OH and H-Asp-Glu-Gly-OH in therapeutically effective quantities for the treatment of the benign prostatic hyperplasia. Yet another aspect of the invention is a method of treatment of the benign prostatic hyperplasia that provides for the administration to a patient of the pharmaceutical composition containing as active components the peptides H-Ala-Asp-Glu-OH, H-Lys-Asp-Glu-OH and H-Asp-Glu-Gly-OH in therapeutically effective quantities.

The effectiveness of the pharmaceutical composition according to the proposed invention was assessed on animals - the model of the benign prostatic hyperplasia caused by testosterone propionate (Example 1).

Table 1 shows the results of a comparative study of the effectiveness of the pharmaceutical composition containing the peptides H-Ala-Asp-Glu-OH, H-Lys-Asp-Glu-OH and H-Asp- Glu-Gly-OH, α ₁-adrenergic blockers (Doxazosin), inhibitor of 5 α-reductase of the 2nd type (Finasteride) and Prostatine, conducted on rats with the benign prostatic hyperplasia. It is clear from the data in Table 1 that the mass of the prostate gland (PG) and the mass factor of the PG (MF, mg/100 r mass of the rat) of intact rats before (102±7 mg and 39±3 respectively) and after the experiment (110±7 mg and 33±2 respectively) were not significantly different.

Compared to intact rats, in control rats testosterone propionate (10 mg/kg/day s/c) administered for 21 days caused a significant (p<0,001) increase of PG - 2.6 times (from 110±7 to 281±20 mg) and the PG MS - 2.4 times (from 33±2 to 80±5).

**Table 1. The influence of the composition of peptides, α₁-adrenergic blocker Doxazosin, inhibitor of 5 α-reductase of the 2nd type Finasteride, and Prostatine on the mass factor (MF) of the prostate gland (PG) in rats with the benign prostatic hyperplasia (BPH)**

| **Animal group** | **Mass of animals, g** | | **Mass of PG, mg** | |
|---|---|---|---|---|
| | Initial | At end of experiment | Initial | At end of experiment |
| Intact prior to experiment /n=10/ | 270±13 | - | 102±7 | 39±3 |
| Intact after experiment /n=11/ | 258±11 | 332±12 | 110±7 | 33±2 |
| Control - modeling of BPH (testosterone s/c + isotonic solution of sodium chloride i/m) /n=10/ | 268±7 | 350±8 | 281±20 | 80±5 |
| BPH + composition (ratio of peptides 1:1:1) (150 mcg/kg/d) i/m /n=10/ | 258±8 | 315±9 | 214±17 | 69±7 |
| BPH + composition (ratio of peptides 1:1:1) (600 mcg/kg/d) i/m /n=10/ | 254±8 | 325±10 | 157±12 | 49±4 |
| BPH + composition (ratio of peptides 1:1:1) (50 mcg/kg/d) i/m /n=10/ | 250±7 | 349±8 | 280±15 | 78±7 |
| BPH + Doxazosin (1 mg/kg/day) in stomach /n=10/ | 251±6 | 311±5 | 238±18 | 77±7 |
| BPH + Finasteride (10 mg/kg/day) in stomach /n=10/ | 256±11 | 312±9 | 199±14 | 65±5 |
| BPH + Prostatine (5 pellets/day) in stomach /n=10/ | 257±8 | 326±9 | 269±21 | 83±7 |

| | | | | |
|---|---|---|---|---|
| Note. The differences are statistically significant (Student's criterion): p<0,05; or p<0,01; or p<0,001. | | | | |

The composition in the dosage of 150-mcg/kg/day i/m for 18 days (starting with day 4 of the experiment) significantly (p<0,05) reduced the mass of the PG, 1.3 times (to 214±17 mg), but the MF PG was not significantly different from the control. In the dosage of 600-mcg/kg/day i/m for 18 days the composition produced a more pronounced effect - significantly (p<0,001) reducing the mass of the PG, 1.8 times (to 157±12 mg) and the MF PG -1.6 times (to 49±4).

Compared to the control, Doxazosin in the dosage of 1 mg/kg/day in the stomach for 18 days did not significantly change either the mass of the PG (reduced just to 238±18 mg) or the MF PG (virtually unreduced). In addition, the animals demonstrated such side effects as diarrhea and torpidity (due, in all probability, to a drop in systemic arterial pressure).

Compared to the control, Finasteride in the dosage of 10 mg/kg/day in stomach for 18 days significantly (p<0,05) reduced the mass of the PG, 1.4 times (to 199±14 mg) and the MF PG -1.2 times (to 65±5).

Compared with the control, the homeopathic remedy Prostatine in the dosage of 5 pellets/day in stomach for 18 days failed to produce and effect on either the mass of the PG (insignificant reduction from 281±20 to 269±21 mg) or the MF PG (increase from 80±5 to 83±7).

As regards the reduction of the mass of the PG, the peptide composition (600 mcg/kg/day) demonstrated a significantly (p<0,01) stronger effect surpassing Doxazosin 1.5 times and Finasteride 1.3 times. In terms of its effect on the MF PG the peptide composition (600 mcg/kg/day) also significantly (p<0,05) surpassed Doxazosin and Finasteride - 1.6 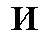 1.3 times respectively.

It follows that testosterone propionate causes an increase in the mass of the PG and MF PG in rats compared to intact animals (development of BPH). Compared to the control, the peptide composition (600 mcg/kg/day) and Finasteride (10 mg/kg/day) reduce the mass of the PG and the MS PG, but the peptide composition surpasses Doxazosin and Finasteride in terms of how pronounced its effect is.

The results of morphological confirmation of the effect of the pharmaceutical composition according to the invention on the prostatic glad of rats on the model of benign prostatic hyperplasia caused by testosterone propionate are shown in Tables 4-8.

The material was subjected to standard histological processing in accordance with Example 2. Sections were stained according to Van Gieson, as well as with hematoxyline and eosin. In addition to the morphological description of the preparations, a quantitative analysis of the most important morphological parameters of the PG (Table 2) to assess the dynamics of morphological changes. The prostate's functional status (fibrosis, epithelium hyperplasia, retention of the prostatic fluid in acinuses) can be assessed using the epithelium/stroma and epithelium/duct indices.

**Table 2. Classes of morphological analysis**

| **Name** | **Characteristic** |
|---|---|
| EPIT | Volume fraction of epithelium, % |
| LUMEN | Volume fraction of acinar lumen, % |
| STROMA | Volume fraction of stroma, % |
| EPIT/STR | Ratio of fractions of epithelium and stroma |
| EPIT/LUMEN | Ratio of fractions of epithelium and acinar lumen |

The morphometric analysis was conducted using a Leica DM2500 microscope fitted with a digital photographic camera. The digital images were used to assess structural changes in PG tissues. Volume fractions of acinuses, stroma and epithelium were defined by point counting. Scoring was used to more accurately determine the quantity of collagen fibers because of their small percentage share (around 1-2%) (Table 3).

**Table 3. The criteria for scoring the quantity of collagen fibers**

| **Score** | **Criterion** |
|---|---|
| 0 | Absent in the field of vision |
| 1 | Thin friable fibers |
| 2 | Thin oriented bunches |
| 3 | Dense interlayers |
| 4 | Zones of fibrosis |

A comparison of the groups in terms of different parameters using the Kruskal-Wallis one-way analysis of variance by ranks, Dan and Wilcoxon-Mann-Whitney test data were obtained on intergroup differences at the set level of significance of p < 0,05.

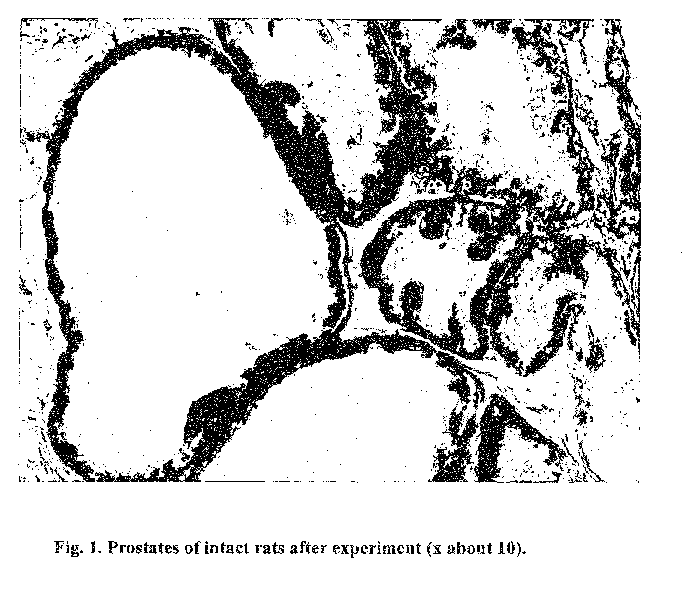

The PG of intact animals is characterized by one-row cubical or high columnar epithelium. The acinuses are partially filled with light fluid and, sometimes, cast-off epithelium. The stroma is moderately friable, and sometimes edemous. The collagen fibers are organized in thin bunches. No pathological changes in the epithelium and stroma were identified (Fig. 1).

The PG of control animals who received testosterone propionate (10 mg/kg/day s/c) for 21 days was significantly different in practically all studied morphometric parameters (except for the collagen content) from that of intact animals (Table. 4).

**Table 4. Changes in morphometric parameters of the prostate gland in rats caused by testosterone propionate at 10 mg/kg subcutaneously for 21 days (control)**

| **Animal group** | **EPIT** | **LUMEN** | **STROMA** | **EPIT/STR** | **EPIT/LUMEN** | **COLL** |
|---|---|---|---|---|---|---|
| **Control** | 29.66±1.11 | 37.70±1.18 | 32.64±1.14 | 90.88±0.89 | 78.67±1.03 | 2 |
| **Intact** | 22.36±1.01 | 40.06±1.19 | 37.58±1.18 | 59.5±1.54 | 55.82±1.56 | 2 |

| | | | | | | |
|---|---|---|---|---|---|---|
| Note. Here and further on in Tables 5-10: EPIT, LUMEN, STROMA - volume fractions of the epithelium, acinar lumens and stroma (%, mean ± standard error of the mean); EPIT/STR - ratio of shares of epithelium to share of stroma; EPIT/LUMEN - ratio of share of epithelium to share of acinar lumens; COLL - score estimate of the content of collagen in the stroma (median). p< 0,05 - the differences are statistically. | | | | | | |

The control animals have a significantly increased share of epithelium, which reflects its enhanced proliferation in response to testosterone. Morphologically, this is expressed in prolific growth of pappilate epithelium projections. Accordingly, the epithelium/stroma ratio is significantly increased. There is also focal fibrosis of the stroma that could not be taken into account because of the insignificant size of the foci.

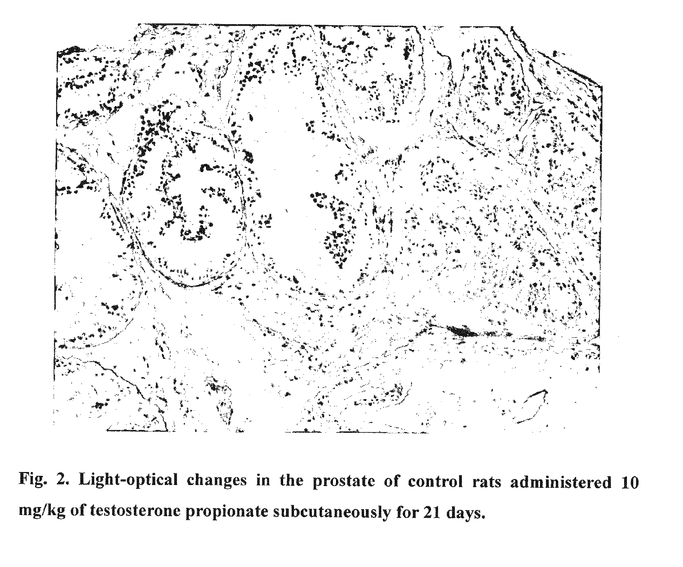

Consequently, the control animals demonstrate signs of the BPH (hyperandrogenism) that includes above all epithelium hyperplasia (Fig. 2).

The animals who received the pharmaceutical composition according to the invention in the dosage of 150 mcg/kg/day i/m for 18 days demonstrated a verifiably smaller share of epithelium that the control group, and the share was not different from that of the intact animals (Table 5).

**Table 5. Changes in the morphometric parameters of the prostate gland of rats with BPH induced by the composition according to the invention in the dose of 50 and 150 mcg/kg/day (intramuscularly for 18 days)**

| **Animal group** | **EPIT** | **LUMEN** | **STROMA** | **EPIT/STR** | **EPIT/LUMEN** | **COLL** |
|---|---|---|---|---|---|---|
| **Composition (ratio of peptides 1:1:1 (150 mcg/kg/day)** | 18.96 ± 0.79 | 59.58 ± 1.00 | 22.29 ± 0.84 | 85.05 ± 1.13 | 31.81 ± 1.07 | 1 |
| **Composition (ratio of peptides 3:1:6) (150 mcg/kg/day)** | 17.50 ± 0.63 | 57.60 ± 0.95 | 23.14 ± 0.95 | 84.25 ± 0.99 | 30.21 ± 1.00 | |
| **Composition (ratio of peptides 2,5:5:2,5) (150 mcg/kg/day)** | 20.18 ± 0.50 | 60.24 ± 0.52 | 21.12 ± 0.54 | 86.04 ± 0.84 | 30.32 ± 1.01 | 1 |
| **Composition (ratio of peptides 1:1:1) (50 mcg/kg/day)** | 15.88 ± 0.54 | 43.17 ± 0.97 | 36.27 ± 0.81 | 62.05 ± 1.14 | 51.07 ± 1.02 | 1 |
| **Control** | 29.66 ± 1.11 | 37.70 ± 1.18 | 32.64 ± 1.14 | 90.88 ± 0.89 | 78.67 ± 1.03 | 2 |
| **Intact** | 22.36 ± 1.01 | 40.06 ± 1.19 | 37.58 ± 1.18 | 59.5 ± 1.54 | 55.82 ± 1.56 | 2 |

| | | | | | | |
|---|---|---|---|---|---|---|
| Note. Here and further on in tables 6-9: p<0,05 - the differences are statistically significant. | | | | | | |

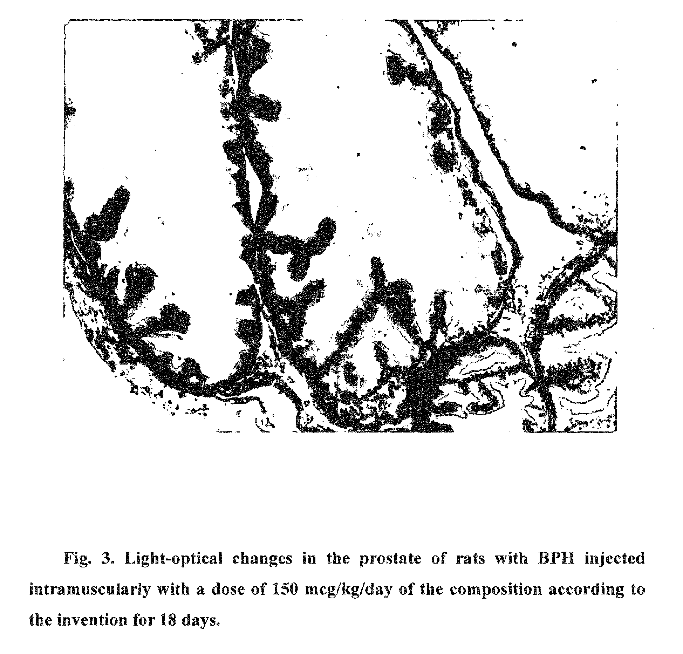

Morphologically, the epithelium does not demonstrate signs of pathology, and its intensive epithalaxia may point to its normalization after the hyperplasia caused by hyperandrogenism. The share of arcinal lumens is significantly greater that in the control group, which points to the dilation of the acinuses. The share of stroma and collagen content in the group is verifiably greater than in intact and control animals, though signs of its inflammation were observed in some cases (dilated full-blood vessels, edema of the stroma), but the inflammation infiltrate was absent. Because of the small size of the stroma the epithelium/stroma ratio in the group is verifiably greater than in the other groups. The verifiably reduced epithelium/acinar lumen index points to the dilation of the acinuses (Fig. 3).

Therefore, the group of animals who received the pharmaceutical composition according to the invention (150 mcg/kg/day) demonstrated, against the background of hyperandrogenism, normalization of the structure of epithelium, the absence of fibrosis, and the dilation of the acinar lumen, with possible retention of the secretion.

In the group of animals who received the pharmaceutical composition according to the invention in a dosage of 600-mcg/kg/day i/m for 18 days, the share of epithelium was verifiably smaller than in the control group, and was not different from that of the intact animals (Table 6).

**Table 6. Transformation of morphometric parameters of the prostatic gland of rats with BPH caused by the pharmaceutical composition according to the invention in the dosage of 600 mcg/kg/day (intramuscularly for 18 days)**

| **Animal group** | **EPIT** | **LUMEN** | **STROMA** | **EPIT/STR** | **EPIT/LUMEN** | **COLL** |
|---|---|---|---|---|---|---|
| **Composition (peptide ratio 1:1:1) (600 mcg/kg/day)** | 22.62± 0.83 | 53.04 ± 0.99 | 24.33 ± 0.84 | 92.97 ± 0.74 | 42.65 ± 1.78 | 2 |
| **Composition (peptide ratio 2:2:6) (600 mcg/kg/day)** | 23.62 ± 0.61 | 52.41 ± 0.64 | 23.11 ± 0.24 | 88.49 ± 0.82 | 40.22 ± 1.00 | 2 |
| **Composition (peptide ratio 2:5:3) (600 mcg/kg/day)** | 22.22 ± 0.51 | 53.05 ± 0.51 | 24.85 ± 0.21 | 93.01 ± 0.15 | 42.85 ± 0.83 | 2 |
| **Control** | 29.66 ± 1.11 | 37.70 ± 1.18 | 32.64 ± 1.14 | 90.88 ± 0.89 | 78.67 ± 1.03 | 2 |
| **Intact** | 22.36 ± 1.01 | 40.06 ± 1.19 | 37.58 ± 1.18 | 59.5 ± 1.54 | 55.82 ± 1.56 | 2 |

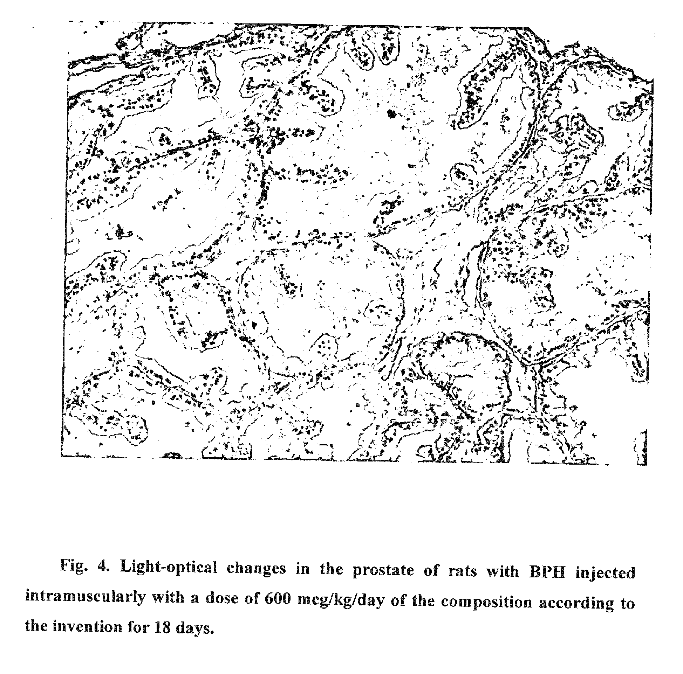

Morphologically the PG's epithelium in this group is not different from the normal one, and some acinuses have the same structure as the intact organ. The external aspect of the secretion was not different from the normal one, too. The fact that the share of acinar lumens was significantly greater than in the above-mentioned groups is due to the heterogeneity of their dimensions, which is probably to the uncompleted normalization of the organ's structure. This is also evidenced by the masses of cast-off epithelium in acinar lumens though its morphology is no different from the intact one. The share of stroma in this group is verifiably smaller that in intact and control animals. The collagen system is less developed than in the control group. The epithelium/stroma and epithelium/acinar lumen ratios in the group are verifiably greater than in intact and control animals, while the epithelium/acinar lumen ratio is small, which points to the dilation of acinar lumens (Fig. 4).

Overall, the PG's morphology in this group is closer to that of the intact group, though the statistical parameters are different from the latter group because of substantial differences in the size of acinar lumens.

The animals that received Doxazosin in a dose of 1 mg/kg/day in stomach for 18 days the total share of epithelium did not differ significantly from the intact norm (Table 7).

**Table 7. Transformation of morphometric parameters of the prostatic gland in rats with BPH caused by Doxazosin in1 mg/kg/day (in stomach for 18 days)**

| **Animal group** | **EPIT** | **LUMEN** | **STROMA** | **EPIT/STR** | **EPIT/LUMEN** | **COLL** |
|---|---|---|---|---|---|---|
| **Doxazosin** | 19.79 ± 0.84 | 54.03 ± 1.05 | 26.19 ± 0.93 | 75.55 ± 1.34 | 36.62 ± 1.19 | 2 |
| **Control** | 29.66 ± 1.11 | 37.70 ± 1.18 | 32.64 ± 1.14 | 90.88 ± 0.89 | 78.67 ± 1.03 | 2 |
| **Intact** | 22.36 ± 1.01 | 40.06 ± 1.19 | 37.58 ± 1.18 | 59.5 ± 1.54 | 55.82 ± 1.56 | 2 |

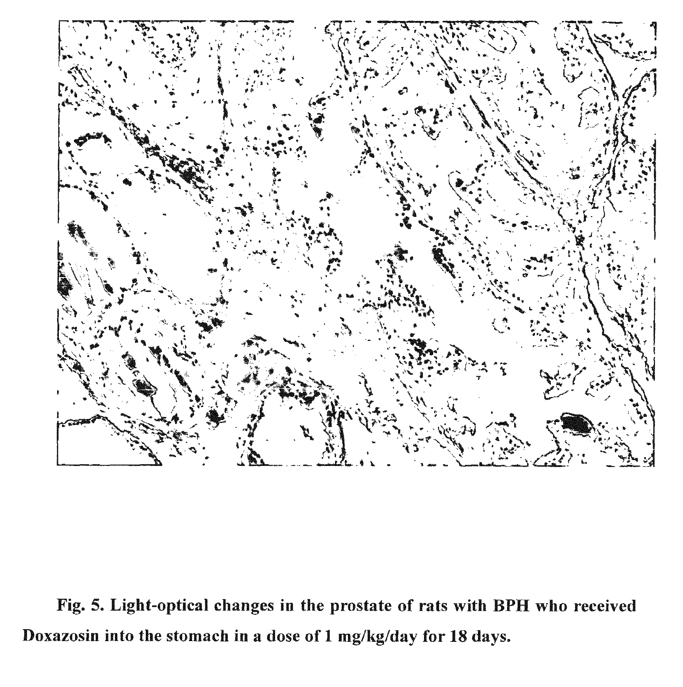

In the Doxazosin group the share of epithelium is verifiably smaller that in the control group and is not different from the share in intact animals, while the acinar lumen is statistically significantly greater than the indicators in the abovementioned groups, which points to the dilation of the acinuses. On the other hand, the share of stroma in the Doxazosin group is verifiably smaller that in intact and control animals, though the content of collagen in all the groups does not differ significantly, while the stroma does not present signs of edema or inflammation. The epithelium/stroma and epithelium/ acinar lumen ratios in the group are verifiably greater than in intact animals but smaller than in the control group. This is apparently related to Doxazosin's pharmacological activity - selective blocking of sub-type 1A alpha₁-andronergic receptors (70% of all sub-types represented in the PG) (Federal Guidelines on the Use of Pharmaceuticals (Formulary System), Issue XII. Eds A.G. Chuchalin, Yu.A. Belousov, V.V. Yasnetsov-M.: Echo, 2011, p. 956; Cohen, Parsons, Combination pharmacological therapies for the management of benign prostatic hyperplasia, Drugs Aging, 2012, Vol. 29, No 4, p. 275-284; Goldfischer E., Kowalczyk J.J., Clark W.R. Hemodynamic Effects of Once-daily Tadalafil in Men With Signs and Symptoms of Benign Prostatic Hyperplasia on Concomitant α(1)-Adrenergic Antagonist Therapy: Results of a Multicenter Randomized, Double-Blind, Placebo-controlled Trial // Urology, 2012, Vol. 79, No 4, p. 875-882), which suppresses hormonal impacts on the PG and slows down its growth (Fig. 5).

Yet the morphological picture of the PG after the influence of Doxazosin is characterized by heterogeneity: large areas of tissue with signs of BPH alternate with dilated acinuses covered by epithelium whose structure is not different from normal. The absence of edema and inflammation in the stroma can be attributed to the positive influence of Doxazosin.

The group of animals who received Finasteride in 10 mg/kg/day in stomach for 18 days presented morphometric parameters that were more similar to those of intact animals than to the control group (Table 8).

**Table 8. Transformation of morphometric parameters of the prostatic gland in rats with BPH under the influence of Finasteride in 10 mg/kg/day (in stomach for 18 days)**

| **Animal group** | **EPIT** | **LUMEN** | **STROMA** | **EPIT/STR** | **EPIT/LUMEN** | **COLL** |
|---|---|---|---|---|---|---|
| **Finasteride** | 20.06 ± 0.95 | 42.40 ± 1.17 | 37.55 ± 1.14 | 53.41 ± 1.55 | 47.31 ± 1.49 | 1 |
| **Control** | 29.66 ± 1.11 | 37.70 ± 1.18 | 32.64 ± 1.14 | 90.88 ± 0.89 | 78.67 ± 1.03 | 2 |
| **Intact** | 22.36 ± 1.01 | 40.06 ± 1.19 | 37.58 ± 1.18 | 59.5 ± 1.54 | 55.82 ± 1.56 | 2 |

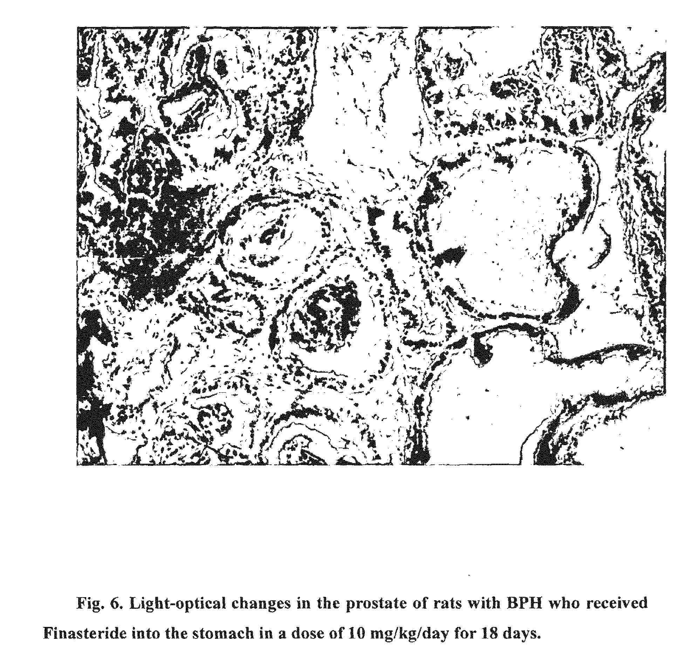

So, the volume fraction of epithelium is significantly smaller than in the control group and is not significantly different for the intact one, which point to the reduction of its proliferation under the influence of Finasteride against the background of hyperandrogenism. This is due to the fact that Finasteride inhibits the enzyme 5 alpha-reductase of predominantly 2nd type and thus blocks the effect of androgens on the PG (Federal Guidelines on the Use of Pharmaceuticals (Formulary System), Issue XII. Eds A.G. Chuchalin, Yu.A. Belousov, V.V. Yasnetsov - M.: Echo, 2011, p. 956; Cohen, Parsons. Combination pharmacological therapies for the management of benign prostatic hyperplasia. Drugs Aging, 2012, Vol. 29, No 4, p. 275-284). However the epithelium's morphology is different from normal (pycnosis and lysis of numerous epithelial cells, and their vacuolization which is probably a sign of apoptosis), which points to its hyperplasia and, apparently, atrophy. Transformation of epithelium's structure is indicated by the nature of the secretion. Although the share of acinar lumens is verifiably not different from the control and intact groups, the prolific vacuolization and the changed color of the secretion are a vicarious indication of its greater viscosity and retention in the acinuses. The epithelium/stroma and epithelium/acinar lumen ratios in this group are significantly smaller than in the control and intact animals. The share of stroma is significantly greater than in the control group. Because the content of collagen in the stroma is verifiably smaller than in the control group, the increase in the share of stroma may be attributed to the edema of the interstitial tissue (Fig. 6). Morphological changes, as compared to the control group, were not observed in all acinuses.

Therefore, the morphological picture of the PG under the impact of Finasteride represents a mosaic of zones with signs of BPH and edemous tissue with atrophic epithelium and acinuses filled with secretion with higher viscosity.

It was discovered that Prostatine has a beneficial effect on the morphology of the PG in 70% (7 out of 10) of the animals. At the same time the volume ratio of epithelium was not different from the intact group and verifiably smaller than in the control group (Table 9). The structure of epithelium was close to normal though acinuses with normal and proliferous epithelium were often located in a mosaic. The share of acinar lumens was much higher than in the intact and control group, an indication of the dilation of the lumens, and there were morphological signs of the secretion's higher viscosity.

**Table 9. Transformation of morphometric parameters of the prostatic gland in rats with BPH under the influence of Prostatine in 5 pellets/day (in stomach for 18 days)**

| **Animal group** | **EPIT** | **LUMEN** | **STROMA** | **EPIT/STR** | **EPIT/LUMEN** | **COLL** |
|---|---|---|---|---|---|---|
| **Prostatine** | 23.68 ± 0.81 | 51.33 ± 0.95 | 24.99 ± 0.83 | 94.75 ± 0.61 | 46.13 ± 1.10 | 1 |
| **Control** | 29.66 ± 1.11 | 37.70 ± 1.18 | 32.64 ± 1.14 | 90.88 ± 0.89 | 78.67 ± 1.03 | 2 |
| **Intact** | 22.36 ± 1.01 | 40.06 ± 1.19 | 37.58 ± 1.18 | 59.5 ± 1.54 | 55.82 ± 1.56 | 2 |

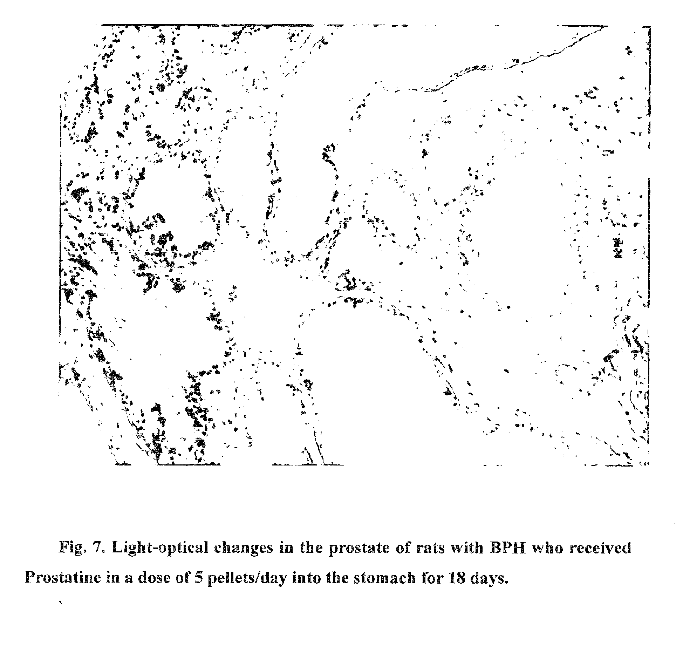

The share of stroma is smaller than in the control and intact groups. The stroma is friable, edemic, and sometimes contains a small amount of infiltrate. The content of collagen is also smaller than in the said groups. As a consequence, the epithelium/stroma ratio is significantly greater, and the epithelium/acinar lumen index is verifiably smaller than in the control and intact groups (Fig. 7).

Therefore, the prostate of rats with BPH who received Prostatine was characterized by partial normalization of the structure of epithelium and signs of retention of the secretion and inflammation phenomena in the stroma.

To sum up the results, it should be noted that epithelium was hyperplastic, which is characteristic of BPH. In all the studied experimental groups the share of epithelium was smaller than in the control group and were not verifiably different from the share in the intact group (Table 10).

**Table 10. The influence of the compositions of peptides, Doxazosin< Finasteride and Prostatine on the volume fraction of epithelium, acinar lumen and stroma of the prostatic gland of rats with BPH**

| **Animal group** | **EPIT** | **LUMEN** | **STROMA** |
|---|---|---|---|
| Intact after experiment | 22.36±1.01 | 40.06±1.19 | 37.58±1.18 |
| Control - modeling of BPH | 29.66±1.11 | 37.70±1.18 | 32.64±1.14 |
| (testosterone s/c + isotonic solution of sodium chloride i/m) | | | |
| BPH + composition | 15.88 ± 0.54 | 43.17 ± 0.97 | 36.27 ± 0.81 |
| (50 mcg/kg/day) i/m | | | |
| BPH + composition | 18.96±0.79 | 59.58±1.00 | 22.29±0.84 |
| (150 mcg/kg/day) i/m | | | |
| BPH + composition | 22.62±0.83 | 53.04±0.99 | 24.33±0.84 |
| (600 mcg/kg/day) i/m | | | |
| BPH + Doxazosin | 19.79±0.84 | 54.03±1.05 | 26.19±0.93 |
| (1 mg/kg/day) in stomach | | | |
| BPH + Finasteride | 20.06±0.95 | 42.40±1.17 | 37.55±1.14 |
| (10 mg/kg/day) in stomach | | | |
| BPH + Prostatine | 23.68±0.81 | 51.33±0.95 | 24.99±0.83 |
| (5 pellets/day) in stomach | | | |

| | | | |
|---|---|---|---|
| Note. The differences are statistically significant: p≤0,05. | | | |

The morphology of epithelium was different in all of those groups. The worst condition of epithelium was in the Finasteride group where the preparation caused injury and death of epithelial cells. In the Prostatine and Doxazosin groups acinuses covered with epithelium with normal morphology alternated with acinuses with hyperplastic epithelium.

In the groups with the composition according to the invention (150 mcg/kg/day and 600 mcg/kg/day) the structure of epithelium did not differ from normal.

In all the groups, except for Finasteride, the volume fraction of acinar lumens was significantly greater that in intact and control animals, amounting to 51-59%, which points to the dilation of the acinar lumens and possible retention of the secretion (Table 10). However, the groups with Prostatine, Doxazosin and the composition according to the invention (150 mcg/kg/day) displayed morphological signs of greater secretion viscosity of varying degrees of intensity (vacuolization, change in color), while in the group of the composition according to the invention (600 mcg/kg/day) even the dilated acinuses contained secretion which was morphologically similar with that in intact animals, which points to possible absence of its retention.

In all the groups, except Finasteride, the volume fraction of stroma was significantly greater than in intact and control animals, amounting to 22-25% (Table 10). Nevertheless, the morphological picture of stroma was noticeably different in different groups. In the Finasteride group the stroma was highly edemous, the signs of edema and inflammation were weaker in the Prostatine group. Inflammation and edema were completely absent in the Doxazosin group, but there were zones of fibrosis. Fibrosis was absent in the group of the composition according to the invention (150 mcg/kg/day), but there were signs of edemous stroma. In the group of the composition according to the invention (600 mcg/kg/day) the stroma was edemous in places, and in some zones there was inflammatory infiltrate in small quantities.

The amount of collagen was verifiably smaller than in the control group, the groups with Prostatine, Finasteride, and the composition according to the invention (150 mcg/kg/day and 600 mcg/kg/day) (but not with Doxazosin), which points to a reduction or absence of fibrosis under the influence of these preparations.

Therefore, based on the totality of morphological and morphometric indicators the structure and function of the PG are restored under the influence of the composition according to the invention preferably in the dose of 150 mcg/kg/day and less preferably in the dose of 600 mcg/kg/day.

Prostatine exerts beneficial influence on the PG's morphology in 70% of cases but its action does not extend to all acinuses, which cannot but affect the organ's function.

Doxazosin also partially normalized the structure of epithelium, and stroma did not display signs of inflammation, but focal fibrosis was observed after its application.

Finasteride removed hyperplasia of the PG's epithelium but exerted damaging actin on it, noticeably worsening the organ's functioning.

It follows that in rats with modeled benign prostatic hyperplasia (BPH) caused be testosterone propionate (10 mg/kg subcutaneously for 21 days), the composition in the dose of 50 mcg/kg/day, 150 mcg/kg/day and 600 mcg/kg/day intramuscularly for 18 days reduces the mass and the mass factor of the prostate gland (PG). At the same time in terms of expressiveness of its action the composition according to the invention is superior to the alpha₁-adrenergic blockers Doxazosin and Finasteride. The known homeopathic medicine Prostatine (5 pellets/day in stomach) has virtually no effect on either the mass or the mass factor of the PG.

The effectiveness of the composition according to the invention in rats with that model of BPH is also confirmed by morphological studies: based on the totality of morphological and morphometric indicators the composition (50 mcg/kg/day, 150 mcg/kg/day and 600 mcg/kg/day) produces the best restoration of the structure and function in comparison with the known drugs. Doxazosin partially normalizes the structure of the PG's epithelium; signs of inflammation are absent in the stroma, but focal fibrosis in observed after its application. Finasteride removes hyperplasia of the PG's epithelium but exerts damaging action on it, noticeably worsening the organ's functioning. Prostatine has a beneficial effect of the PG's morphology in 70% of rats but its action does not extend to all acinuses.

The composition according to the invention in the dose of 6 mg/kg (one-time abdominally) does not provoke during 14 days significant toxic reactions in rats.

The composition according to the invention can be produced in the form of pills, and capsules using pharmaceutically acceptable excipients and methods traditional for this industry, it can also be produced in the form of solution for injection. In addition, the composition can be manufactured in the form of drops or solution for oral use, or nasal spray or drops, subglossal or oral aerosol, or a powder, or a lyophilisate for preparation of solution.

The abovementioned forms of the pharmaceutical composition are obtained with well-known methods. The composition according to the invention is administered perorally or parentereally (e.g., intravenously, intramuscularly, subcutaneously). Any pharmaceutically acceptable media or solutions can be used to obtain the composition according to the invention. The pharmaceutical composition for perioral administration can be in the form of solutions, suspensions, tablets or pills, capsules, powders, and the like. Tablets containing various excipients, e.g. sodium citrate, calcium carbonate and calcium phosphate, or any other pharmaceutically acceptable excipients, are used together with various disintegrators, e.g. starch, preferably potato starch or tapioca, complex silicates or any other pharmaceutically acceptable disintegrators, together with binding excipients, e.g., polyvinyl pyrrolidone, sacharose, gelatin, gummi arabicum, and any other pharmaceutically acceptable binding excipients. In addition, slip agents are frequently used, e.g., magnesium stearate, sodium laurel sulfate, talcum for tabletization, or any other pharmaceutically acceptable substances. The composition according to the invention are also used in soft and hard filled gelatin capsules; in such a case the preferred materials include, for example, lactose or sugar of milk, as well as polyethylenglycols with high molecular weights. If water suspensions and/or elixirs are desired for perioral administration, the compounds according to this invention can be combined with various sweetening agents to improve taste and smell, coloring agents, emulsifying and/or suspending agents, as well as solvents such as water, ethanol, propylene glycol, glycerin, and their various combinations. Solutions in sesame seed oil or peanut oil or in water propylene glycol, as well as sterile water solutions or corresponding water-soluble salts can be used for parenteral administration. If necessary, such water solutions can be buffer ones, and a water solution is first isotonified with the help of a sufficient quantity of a salt or glucose solution. These water solutions are particularly adapted for intravenous, intramuscular, subcutaneous and abdominal introduction. To this end, water media can be easily obtained using standards techniques that are well known to specialists.

The method of treating benign prostate hyperplasia includes prescription of the pharmaceutical composition containing the peptides H-Ala-Asp-Glu-OH, H-Lys-Asp-Glu-OH and H-Asp-Glu-Gly-OH in therapeutically effective quantities.

The preferred embodiment includes prescription of the composition containing the peptides H-Ala-Asp-Glu-OH, H-Lys-Asp-Glu-OH and H-Asp-Glu-Gly-OH in the ratio of (1-8):(1-8):(1-8).

The regimen and dosage of administration of the pharmaceutical composition according to the invention, and the duration of administration depend on the stage of the disease and the patient's condition.

The invention is illustrated with the following examples.

### Example 1.

The effect of the new mixtures of peptides on the mass of the prostate of rats in the model of benign prostate hyperplasia caused by testosterone propionate, was studied on 98 white non-pedigree male rats (mass 220-280 g at the beginning of the experiment), and 10 white non- pedigree male rats with a mass of 22-26 g (acute toxicity was assessed in those animals in a separate series o experiments).

BPH was modeled in the experimental rats for 21 days with subcutaneous injections of testosterone propionate (OAO «Dalkhimfarm», Russia) in the dose of 10 mg/kg; the model has been recommended both by Russian and foreign researchers (Frolov N.Yu., Buriakina A.V., Melnikova T.I. et al. Methodological approaches to experimental study of pharmacological substances for the prevention and treatment of diseases of the prostatic gland. Experimental and clinical pharmacology, 2010, 73, No. 6, pp. 41-44; Bisson J.F., Hidalgo S., Rozan P., Messaoudi M. Therapeutic effect of ACTICOA powder, a cocoa polyphenolic extract, on experimentally induced prostate hyperplasia in Wistar-Unilever rats. J. Med. Food., 2007, Vol. 10, No 4, p. 628-635; Liu H.P., Chen G.L., Liu P., Xu X.P. «Amlodipine alone or combined with terazosin improves lower urinary tract disorder in rat models of benign prostatic hyperplasia or detrusor instability: focus on detrusor overactivity". BJU Int., 2009, Vol. 104, No 11, p. 1752-1757).

The animals in those groups were injected intramuscularly (i/m) a mixture of peptides in doses of 50, 150 and 600 mcg/kg/day, alpha₁-adrenergic blocker Doxazosin (Zoxon, Zentiva a.s., Czech Republic) in the dose of 1 mg/kg/day and 2nd type 5 alpha-reductase inhibitor Finasteride (Proscar, Merck Sharp & Dohme, The Netherlands) in the dose of 10 mg/kg/day in the stomach (via an intragastral sonde) for 18 days (from day 4), and also the well-known homeopathic drug Prostatine (OOO «Homeofarma», Russia) with 5 pellets into the stomach for 18 days. The rats in the control group received intramuscularly only an isotonic solution of sodium chloride (NaCl) in equivolume quantities.

Extraction of the prostatic gland (PG) for morphological (light-optical) analysis was done both before and after the reproduction of BPH (on days 22 and 23). The rats were decapitated under general anesthesia with ether, following which the prostatic gland was extracted and weighed (analytical balance «Sartogosm LV 120-A», Russia, inaccuracy-0.5 mg).

### Example 2. Morphological confirmation of the action of the composition according to the invention on the prostatic gland of rats in the model of benign prostatic hyperplasia induced by testosterone propionate

The material was subjected to standard histological processing. For light-optical analysis, pieces of the PG were fixed with 10% neutral formalin, following which histological sections were prepared. The sections were stained according to Van Gieson, and also with hematoxyline and eosin. In addition to morphological description of the microsections, a quantitative evaluation of the most important morphological parameters of the PG for the purpose of objective assessment of the dynamics of morphological changes. A Leica DM2500 microscope with a digital photographic camera was used for morphometric analysis. The digital images were used to assess structural changes in the PG's tissues.

### Example 3. Determination of acute toxicity of the composition in accordance with the invention.

A separate series of experiments was conducted to determine acute toxicity in 10 white non-pedigree male rats with a mass of 22-26 g. The composition according to the invention was introduced in the dose of 6 mg/kg intraperitoneally (i/p) (this dose is 10 times higher than the maximum effective one). It was established that during 14 days following a one-time i/p injection the composition did not cause either the death of animals or any significant toxic reactions.

Determination of acute toxicity of the peptide composition was conducted on white non-pedigree male rats (during 14 days), with registration of their death (14 days after the one-time intraperitoneal injection of the pharmaceutical composition in accordance with the invention).

StatPlus 2009 Professional, with the use of parametric and non-parametric criteria, was used for statistical processing of the results of the analysis.

The results of the studies indicated therefore that the pharmaceutical composition proposed in accordance with this invention possesses a pronounced effect in regard to benign prostatic hyperplasia, that is considerably superior to the known drugs such as α₁-adrenergic blockers, inhibitors of 5 α-reductase, and the homeopathic drug Prostatine, and, and has no side effects.

## Claims

1. Pharmaceutical composition that is effective in regard to benign prostatic hyperplasia, and distinct in that it contains such active components as the peptides Ala-Asp-Glu, Lys-Asp-Glu and Asp-Glu-Gly in therapeutically effective quantities.

2. Pharmaceutical composition according to claim **1,** containing the peptides Lys-Asp-Glu, Ala-Asp-Glu and Asp- Glu-Gly in the mass ratio of (1-8):(1-8):(1-8).

3. Pharmaceutical composition according to claim **1** in the form of tablets, capsules, nasal stray or nasal drops, subglossal or oral aerosol, perioral drops, a powder, or a solution for injection, or lyophilisate for preparation of solutions.

4. Method of treating benign prostatic hyperplasia distinct in that patients are prescribed the pharmaceutical composition in 1 above in effective quantity.

5. The use of the pharmaceutical composition according to claim **1** for treatment of benign prostatic hyperplasia.
